# EUROPÄISCHE PATENTSCHRIFT

(11) **EP 1 027 058 B1**
(45) Veröffentlichungstag und Bekanntmachung des Hinweises auf die Patenterteilung: **21.12.2005**
(21) Anmeldenummer: 98950037.6
(22) Anmeldetag: 16.09.1998
(51) Int. Cl.: A61K 31/70, A61P 3/10

(54) **DISACCHARIDDERIVATE ZUR BEHANDLUNG VON HYPERGLYKÄMIEN**
DISACCHARIDE DERIVATIVES FOR TREATING HYPERGLYCAEMIA
DERIVES DISACCHARIDIQUES DESTINES AU TRAITEMENT DE L'HYPERGLYCEMIE

(30) Priorität: 31.10.1997 DE 19748195
(43) Veröffentlichungstag der Anmeldung: 16.08.2000
(73) Patentinhaber: Südzucker Aktiengesellschaft Mannheim/Ochsenfurt, 68165 Mannheim (DE)
(72) Erfinder: HEINZ, Fritz, D-30629 Hannover (DE); HERTEL, Sabine, D-30851 Langenhagen (DE); KUNZ, Markwart, D-67550 Worms (DE); VOGEL, Manfred, D-67271 Neuleiningen (DE)
(74) Vertreter: Schrell, Andreas
(86) Internationale Anmeldenummer: PCT/EP1998/005857
(87) Internationale Veröffentlichungsnummer: WO 1999/022740

(56) Entgegenhaltungen:
- EP-A- 0 560 284
- EP-A- 0 599 646
- DE-A- 2 830 424
- DE-A- 4 307 388
- DE-A- 4 310 032
- DE-A- 19 542 303
- GB-A- 2 011 397
- KUNZ M ET AL: "KATALYTISCHE OXIDATION VON ISOMALTULOSE" CHEMIE. INGENIEUR. TECHNIK, Bd. 67, Nr. 7, Juli 1995, Seiten 836-842, XP000513082 in der Anmeldung erwähnt
- M. PIETSCH ET AL: "Regioselective synthesis of new sucrose derivatives via 3-ketosucrose" CARBOHYDRATE RESEARCH, Bd. 254, 17. Februar 1994, Seiten 183-194, XP002093622 in der Anmeldung erwähnt

## Beschreibung

Die Erfindung betrifft Arzneimittel- und Lebensmittelzusammensetzungen zur Behandlung von Hyperglykämien im menschlichen oder tierischen Körper sowie Verfahren zur Inhibierung von α-Glucosidasen.

Der Abbau von Kohlenhydraten im menschlichen oder tierischen Körper setzt das Vorhandensein von α-Glucosidasen, insbesondere von Saccharasen und Maltasen, voraus. Inhibitoren dieser beiden Enzyme erweisen sich insbesondere dann als vorteilhaft, wenn ein Anstieg des Blutzuckerspiegels nach den Mahlzeiten verhindert werden soll. Aus der EP 0 560 284 Al sind α-Glucosidaseinhibitoren bekannt, die ohne schädliche Auswirkungen auf den Körper des Patienten sind und zudem von diesem nur in geringem Ausmaß aufgenommen werden. Aus dieser Druckschrift ist die Verwendung verschiedener Pentosen und Hexosen wie L-Arabinose, L-Fucose, L-Xylose, D-Ribose etc. bekannt, um die α-Glucosidaseaktivität in einem Maltase und Saccharase enthaltenden Homogenat zu inhibieren.

Aus der GB 2 011 397 A1 ist bekannt, daß von Streptomyces (Actinomyces A 2396) produzierte Zucker eine Glycosidase-inhibierende Wirkung zeigen. Bei den in der GB 2 011 397 A1 offenbarten Zuckern handelt es sich um oligomere Verbindungen, die mindestens Trisaccharide sind.

Das der vorliegenden Erfindung zugrundeliegende technische Problem liegt darin, eine einen Inhibitor für die Enzym-Aktivität von Saccharase und Maltase enthaltende Arzneimittel- oder Lebensmittelzusammensetzung bereitzustellen, wobei der Inhibitor eine besonders starke inhibierende Wirkung auf beide genannten, insbesondere auch jede einzelne dieser Enzym-Aktivitäten bei gleichzeitiger guter Körperverträglichkeit aufweist.

Das der vorliegenden Erfindung zugrunde liegende technische Problem wird durch die Bereitstellung der Disaccharidderivate gemäß Patentanspruch 1 zur Behandlung von Hyperglykämien des menschlichen oder tierischen Körpers gelöst.

Das der vorliegenden Erfindung zugrundeliegende technische Problem wird auch durch die Bereitstellung einer Arzneimittel- oder Lebensmittelzusammensetzung gemäß der Patentansprüche gelöst, die Disaccharidderivate, insbesondere oxidierte Disaccharide oder deren Derivate wie Amide oder Alkylester der oxidierten Disaccharide oder Aminoderivate von Disacchariden, sowie gegebenenfalls einen pharmazeutisch verträglichen Träger enthält. In besonders bevorzugter Ausführungsform der Erfindung stellen die erfindungsgemäß vorgesehenen Derivate der oxidierten Disaccharide Methyl- und Ethylester des o-xiderten Disaccharids dar. In einer weiteren bevorzugten Ausführungsform stellen die erfindungsgemäßvorgesehenen Derivate der oxidierten Disaccharide deren Amide mit der allgemeinen Struktur R'-CO-NHR dar, wobei R'-CO das oxidierte Disaccharid und R=H bzw. R= CₙH(₂ₙ₊₁) mit n = 0-5 ist. Insbesondere wird das technische Problem durch eine derartige Arzneimittel- oder Lebensmittelzusammensetzung gemäß der Patentansprüche gelöst, die eine Monocarbonsäure von Saccharose, insbesondere die C6-Saccharosemonocarbonsäure, im folgenden Saccharose-C6-Säure genannt, und/oder eine oxidierte Palatinose (Isomaltulose), insbesondere die Palatinose-C6'-Säure enthält. Das Problem wird auch durch eine Lebensmittel- oder Arzneimittelzusammensetzung gelöst, die in bevorzugter Ausführungsform eine Aminosaccharose enthält, insbesondere die 3'-Aminosaccharose.

Es konnte überraschenderweise gezeigt werden, daß die Enzymaktivitäten von Saccharase und Maltase durch die erfindungsgemäß einzusetzenden vorgenannten Disaccharid-Derivate, insbesondere die 3'-Aminosaccharose) und die oxidierten Disaccharide beziehungsweise deren vorgenannte Alkylester oder Amide, insbesondere die Saccharose-C6-Säure und die Palatinose-C6'-Säure, besonders stark inhibiert wurden. Die erfindungsgemäß eingesetzten Verbindungen eignen sich daher zur Verringerung des Blutzuckerspiegels und demgemäß zur Vorbeugung und Behandlung von Hyperglykämien.

Die Erfindung betrifft auch eine Arzneimittelzusammensetzung, enthaltend als aktiven Bestandteil die erfindungsgemäß einzusetzenden vorgenannten Disaccharidderivate, insbesondere 3'-Aminosaccharose, und/oder oxidierte Disaccharide oder deren vorgenannte Alkylester oder Amide, insbesondere Sacchararose-C6-Säure und/oder Palatinose-C6' -Säure, und einen pharmazeutisch verträglichen Träger oder Zusatzstoffe zur Verringerung eines erhöhten Zuckerspiegels bzw. zur Behandlung und Vorbeugung von Hyperglykämien des tierischen und menschlichen Körpers. Die erfindungsgemäße Arzneimittelzusammensetzung kann demgemäß sowohl prophylaktisch als auch therapeutisch eingesetzt werden.

Die Erfindung betrifft auch eine Lebensmittelzusammensetzung bzw. sogenanntes "functional food", enthaltend ein Aminoderivat eines Disaccharids, ein Amid eines oxidierten Disaccharids mit der allgemeinen Strukturformel R' -CO-NHR, wobei R'-CO das oxidierte Disaccharid und R=H beziehungsweise R=CₙH₍₂ₙ₊₁₎ mit n=0-5 ist, ein Methyl- oder Ethylester des oxidierten Disaccharids oder Palatinose-C6'-Säure sowie gegebenenfalls lebensmittelverträgliche Zusatzstoffe, die bzw. das beispielsweise zur Vorbeugung oder begleitenden Behandlung von erhöhtem Blutzuckerspiegel und Hyperglykämien eingesetzt werden kann.

Die erfindungsgemäß eingesetzten, vorstehend beschriebenen Disaccharidderivate weisen den Vorteil auf, daß sie eine stark inhibierende Wirkung auf die Saccharase- und Maltaseaktivität ausüben. Insbesondere eignen sich die erfindungsgemäß eingesetzten. Disaccharidderivate daher zur Herstellung eines Arzneimittels und/oder Lebensmittels zur Vorbeugung oder Behandlung von Hyperglykämien.

Die Erfindung betrifft auch die Verwendung der er-Eindungsgemäß eingesetzten vorstehend beschriebenen Disaccharidderivate zur Herstellung der vorgenannten Zusammensetzungen gemäß der Patentansprüche.

Pharmazeutisch und/oder lebensmittel-verträgliche in Verbindung mit den erfindungsgemäß eingesetzten Disaccharidderivaten einsetzbare Träger oder Zusatzstoffe sind zum Beispiel Bindemittel, Konservierungsmittel, Stabilisatoren, Trenn- und Gleitmittel, Süßstoffe; Farb- und Aromastoffe oder ähnliches.

Die erfindungsgemäßen Zusammensetzungen können als Tabletten, Pulver, Pillen, Komprimate oder Lösungen vorliegen und beispielsweise oral oder intraperitoneal verabreicht werden. Die Dosierung beträgt vorzugsweise 0,2 bis 90 Gew.-%.

In erfindungsgemäßen Lebensmittelzusammensetzungen ist vorzugsweise vorgesehen, mindestens 0,2 Gew.-%, vorzugsweise 2 bis 50 Gew. -% (bezogen auf das Gesamtgewicht im Lebensmittel enthaltender Kohlenhydrate) an den erfindungsgemäßen Disacharidderivaten einzusetzen.

Die Erfindung betrifft also auch ein in vitro-Verfahren zur Inhibierung von α-Glucosidasen gemäß Patentanspruch 11, insbesondere von Maltase- und Saccharaseaktivitäten, wobei eine wässrige; den Glucoamylase/Maltase-Enzymkomplex und/oder den Saccharase/Isomaltase-Enzymkomplex enthaltende Lösung mit den erfindungsgemäß einzusetzenden Disaccharidderivaten, insbesondere der 3'-Aminosaccharose, den oxidierten Disacchariden oder deren Alkylestern oder Amiden, insbesondere der Saccharose-C6-Säure und/oder Palatinose-C6'-Säure versetzt und eine wirksame Inhibierung der Saccharase- und/oder Maltaseaktivität erreicht wird.

Die Erfindung wird anhand eines Ausführungsbeispiels näher erläütert.

### Beispiel:

Bestimmung der Enzymaktivitäten von Saccharase, Maltase und Isomaltase in den Enzymkomplexen Saccharase/Isomaltase (SI) und Glucoamylase/Maltase (GM)
A) Die Aktivität des SI Enzymkomplexes wurde mit drei verschiedenen Substraten, nämlich Saccharose, Maltose und Isomaltose untersucht.
Die Isomaltaseaktivität wurde mit dem Substrat Isomaltose untersucht.
Die Maltaseaktivität wurde mit dem Substrat Maltose untersucht.
Die Herstellung der 3'-Aminosaccharose ist in Pietsch, M. et al., Carbohydrate research 254 (1994), 183 bis 194, beschrieben.
Die Herstellung der Palatinose-C6'-Säure ist in Kunz et al., Chem.-Ing.-Tech. 67 (1995), 836-842 beschrieben.
Die Herstellung der Saccharose-C6-Säure ist in der EP 0 651 734 B1 beschrieben.
Triethanolamin-Hydrochlorid (TRA), Adenosin-5'-triphosphat (ATP), Nicotinamid-adenindinukleotid (NAD), Hexokinase (HK) und Glucose-6-phosphat-Dehydrogenase (G6PDH) wurden von der Firma Boehringer/Mannheirn sowie Magnesiumchlorid-Hexahydrat von der Firma Merck bezogen.
Die Saccharase/Isomaltase (SI) und die Glucoamylase/Maltase (GM) wurden mittels Papainabdauung aus Schweinedünndarm-Mukosa isoliert und durch Ammoniumsulfatfällung angereichert. Die Auftrennung der Enzyme in isolierte Saccharase/Isomaltase (SI) und Glucoamylase/Maltase (GM) erfolgte über einen Ionenaustauscher (DEAE-Cellulose), sowie eine sich anschließende Feinauftrennung mittels Gelfiltration auf einer Superdex 200-Säule der Firma Pharmacia.
B) In den im folgenden beschriebenen Verfahren zur Bestimmung der Enzymaktivität der Saccharase/Isomaltase und Glucoamylase/Maltase unter dem Einfluß der untersuchten Substanzen wurde als Meßgröße die freigesetzte Glucose bzw. Fructose bestimmt. Dies geschieht im enzymatisch-optischen Test mit Hexokinase (HK) und Glucose-6-phosphat-Dehydrogenase (G6PDH) als gekoppeltem Nachweissystem (Beutler, H.-O.; in: Methods of Enzymatic Analysis; Bergmeyer, H.U.; Bergmeyer, J.; Graßl, M. (Herausgeber); 3. Ausgabe; Vol. VI, 2-10). Die Messung erfolgte bei einer Temperatur von 37°C und einem pH-Wert von 7,0.
Die folgende Tabelle verdeutlicht die Zusammensetzung der für die einzelnen Untersuchungen notwendigen Ansätze. Bei einzelnen Untersuchungen wurde der Halbmikro-Ansatz verwendet, der jedoch in einigen Fällen auf einen Mikro-Ansatz reduziert wurde.

**Tabelle 1:**

| Zusammensetzung der einzelnen Ansätze | | | | |
|---|---|---|---|---|
| Halbmikro-Ansatz | | | Mikro-Ansatz | |
| Substanz¹⁾ | Volumen | Konzentration im Ansatz | Substanz¹⁾ | Volumen |
| Substrat-lösung (1,6*X mM) | 0,620 mL | X mmol/L, variierend innerhalb einer Meßreihe | Substrat-lösung (1,67 *X mM) | 0,210 mL |
| TRA (100 mM), pH 7 | 0,100 mL | 100 mmol/L | TRA (100 mm), pH 7 | 0.035 mL |
| Mischung aus: | 0,250 mL | | Mischung aus: | 0,075 mL |
| (i) ATP (16,2 mM) | | 4,05 mmol/L | (i) ATP (18,9 mM) | |
| (ii) NAD⁺ (4,4 mM) | | 1,1 mmol/L | (ii) NAD⁺ (5,1 mM) | |
| (iii) MgCl₂ (40 mM) | | 10 mmol/L | (iii) MgCl₂ (46,7 mM) | |

| Ansatz innerhalb von 15 min auf 37°C temperieren | | | | |
|---|---|---|---|---|
| Mischung aus: | 0,010 mL | | Mischung aus: | 0,010 mL |
| (i) HK (70 U/mL) | | 0,7 U/mL | (i) HK (24,5 U/mL) | |
| (ii) G6P-DH (500 U/mL) | | 5,0 U/mL | (ii) G6P-DH (175 U/mL) | |

| im Substrat enthaltene Glucose abreagieren lassen; dazu weitere 5 min inkubieren | | | | |
|---|---|---|---|---|
| Enzym (5 U/mL Maltase-aktivität) | 0,020 mL | 0,1 U/mL | Enzym (1,75 U/mL Maltase-aktivität) | 0,020 mL |
| 1,000 mL | | | 0,350 mL | |

| | | | | |
|---|---|---|---|---|
| ¹⁾Alle Lösungen wurden in TRA-Puffer (100 mM), pH 7, angesetzt. | | | | |

C) Bevor die Inhibitorstärken bestimmt wurden, wurde für jede Enzym-Substrat-Kombination eine Grundkinetik erstellt, bei der sowohl die Michaelis-Menten-Konstante (K_{M} [mmol/L]) als auch die maximale Umsatzgeschwindigkeit (Vₘₐₓ [µmol/(mLmin]) bestimmt wurden. Für die Enzym/Substrat-Kombination SI/Saccharose wurde eine Substrat-Konzentration von 1,24 bis 124 mmol/L, für die Enzym/Substrat-Kombination SI/Maltose wurde eine Substrat-Konzentration von 0,62 bis 24,8 mmol/L, für die Enzym/Substrat-Kombination SI/Isomaltose wurde eine Substrat-Konzentration 3,6 bis 57,6 mmol/L und für die Enzym/Substrat-Kombination GM/Maltose wurde eine Substrat-Konzentration von 0,31 bis 12,4 mmol/L im Ansatz verwendet.

**Tabelle 2:**

| Kinetische Konstanten der SI für die Substrate Saccharose, Maltose und Isomaltose und der GM für Maltose als Substrat | | |
|---|---|---|
| Enzym/Substrat-Kombination | K_{M} [mmol/L] | Vₘₐₓ [U/mL] |
| SI/Saccharose | 58,8 ± 11,4 | 7,7 ± 1,7 |
| SI/Maltose: | 11,2 ± 2,6 | 5.9 ± 0,4 |
| SI/Isomaltose: | 73,9 ± 6,8 | 3,07 ± 0,18 |
| GM/Maltose: | 4,2 ± 1,0 | 4,8 ± 0,7 |

Die Tabelle 2 stellt die K_{M}- und Vₘₐₓ-Werte der Saccharase/Isomaltase (SI) mit den Substraten Saccharose, Maltose und Isomaltose und der Glucoamylase/Maltase (GM) mit dem Substrat Maltose dar (angegeben werden die Mittelwerte aus allen durchgeführten Grundkinetiken, der Vₘₐₓ-Wert bezieht sich auf eine Enzym-Lösung mit einer Maltaseaktivität von 5 U/mL, bestimmt bei Substratsättigung).
D) Zunächst wurde untersucht, inwieweit die erfindungsgemäßen Disaccharidderivate und Vergleichssubstanzen, nämlich Saccharose und Maltose, durch SI bzw. durch die GM bei Inkubation für 24 Stunden gespalten wurden. 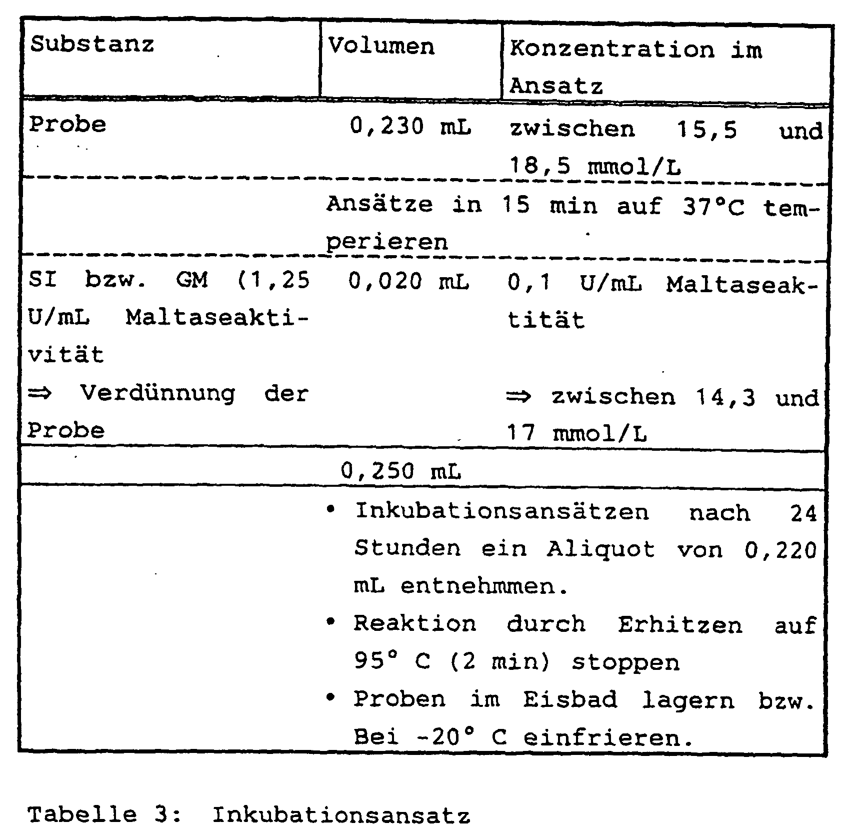
Die Ergebnisse sind in der Tabelle 4 dargestellt.

**Tabelle 4:**

| Spaltung der Derivate von Disacchariden bei Inkubation (24 Stunden) mit SI bzw. GM im Vergleich zur Spaltung der natürlichen Substrate Saccharose und Maltose | | | |
|---|---|---|---|
| Substanz | nachgewiesenes Spaltprodukt | Umsatz durch SI [%] | Umsatz durch GM [%] |
| Saccharose | Glucose | 85 | keine Inkubation durchgeführt |
| | Fructose | 87 | |
| Maltose | Glucose | 100 | 95,7 |
| 3'-A-Sacch | Fructose | 0 | 0 |
| Sacch-C6S | Glucose | 2 | 0 |
| Pal-C6'-S | Fructose | 0,4 | 0,1 |
| (3'-A-Sacch: 3'-Aminosaccharose, Sacch-C6S: Saccharose-C6-Säure, Pal-C6'S: Palatinose-C6'-Säure) | | | |

E) Zu jeder Enzym-Substrat-Kombination wurden anschließend Inhibitorkinetiken. mit 3'-Amino-saccharose, Saccharose-C6-Säure und Palatinose-C6,-Säure durchgeführt. Die Höhe der eingesetzten Inhibitorkonzentrationen richtet sich nach der Stärke der resultierenden Hemmungen. Für jede der Enzym-Substrat-Kombinationen wurden 3 bis 4 Kinetiken mit verschiedenen Inhibitorkonzentrationen erstellt. Die Inhibitorkonstanten Kᵢ und Kᵢᵢ wurden, wenn nicht anders angegeben, aus den Sekundärplots der Lineweaver-Burk-Darstellung bestimmt.
In den folgenden Tabellen steht K für kompetitive, NK für nichtkompetitive und UK für unkompetitive Hemmung.

**Tabelle 5:**

| Inhibierung von SI/Saccharose und SI/Maltose durch die 3'Amino-Saccharose. Vergleich (i) der Inhibierung bei zwei verschiedenen Vorgehensweisen und (ii) der Kᵢ-Werte, die sich aus einer Henderson- bzw. Dixon-Auswertung ergeben. | | | | | | |
|---|---|---|---|---|---|---|
| | ohne Vorinkubation | | | mit Vorinkubation | | |
| | Typ | Kᵢ [* 10⁻⁶ M] | | Typ | Kᵢ [* 10⁻⁶ M] | |
| SI/Saccharose | NK | Henderson | 5,0 | K | Henderson | 6,3 |
| | | Dixon | 5,2 | | Dixon | 6,5 |
| SI/Maltose | NK | Henderson | 5,2 | K | Henderson | 4,6 |
| | | Dixon | 5,4 | | Dixon | 4,6 |

**Tabelle 6:**

| Inhibierung der durch SI katalysierten Hydrolyse von Saccharose, Maltose und Isomaltose durch Carboxyderivate von Saccharose und Palatinose | | | | | | | | | |
|---|---|---|---|---|---|---|---|---|---|
| Carboxy-derivat | SI/Saccharose | | | SI/Maltose | | | SI/Isomaltose | | |
| | Typ | Kᵢ [mm] | Kᵢᵢ [mm] | Typ | Kᵢ [mM] | Kᵢᵢ [mM] | Typ | Kᵢ [mM] | Kᵢᵢ [mm] |
| Sacch-C6S | NK | 9 | 9 | NK | 43 | 5 | NK | 73 | 3 |
| Pal-C6'S | NK | 7 | 8 | NK | 16 | 31 | K | 20 | - |

**Tabelle 7:**

| Inhibierung der durch GM katalysierten Hydrolyse von Maltose durch 3'-Aminosaccharose | | | |
|---|---|---|---|
| Aminoderivat | Typ | Kᵢ [mM] | Kᵢᵢ [mM] |
| 3'A-Sacch | NK | 1 | 17 |

**Tabelle 8:**

| Inhibierung der durch GM katalysierten Hydrolyse von Maltose durch Carboxyderivate von Saccharose und Palatinose | | | |
|---|---|---|---|
| Carboxyderivat | Typ | Kᵢ [mM] | Kᵢᵢ [mM] |
| Sacch-C6S | UK | - | 4 |
| Pal-C6'S | K | 20 | - |

Es zeigt sich, daß die 3'-Aminosaccharose die Enzymkomplexe SI und GM bereits in sehr niedrigen Konzentrationen zu einem hohen Prozentsatz inhibiert (Tabellen 4 und 6). Im Fall der Kombination SI/Saccharose und SI/Maltose war bei einer Inhibitorkonzentration von 1 mmol/L bereits keinerlei enzymatische Aktivität mehr meßbar. Bei einer Konzentration von 5 µmol/mL wurde die Saccharosehydrolyse zu etwa 30% und die Maltosehydrolyse zu etwa 50% inhibiert.
Es zeigt sich auch, daß die erfindungsgemäß eingesetzten Carboxyderivate von Saccharose und Palatinose sowohl die SI als auch die GM deutlich inhibieren (Tabellen 6 und 8).
F) Die folgenden Tabellen stellen Übersichten über die Hemmstärken dar, mit denen die erfindungsgemäß eingesetzten Disaccharidderivate auf die untersuchten Enzym-/Substrat-Kombinationen einwirken.

**Tabelle 9:**

| Inhibierung von SI durch Carboxyderivate von Saccharose und Palatinose: K_{M}/Kᵢ-Werte sowie die Verhältnisbildung von kompetitiven und unkompetitiven Hemmanteilen | | | | | | | | | |
|---|---|---|---|---|---|---|---|---|---|
| Carboxyderivat | SI/Saccharose | | | SI/Maltose | | | SI/Isomaltose | | |
| | Typ | K_{M}/Kᵢ | Kᵢ/Kᵢᵢ | Typ | K_{M}/Kᵢ | Kᵢ/Kᵢᵢ | Typ | K_{M}/Kᵢ | Kᵢ/Kᵢᵢ |
| Sacch-C6S | NK | 6,2 | 1 | NK | 0,26 | 9 | NK | 1 | 21,6 |
| Pal-C6'S | NK | 8,4 | 0,88 | NK | 0,7 | 0,54 | K | 3,7 | - |

**Tabelle 10:**

| Inhibierung von GM durch Carboxyderivate von Saccharose und Palatinose: K_{M}/Kᵢ-Werte sowie die Verhältnisbildung von kompetitiven und unkompetitiven Hemmanteilen | | | |
|---|---|---|---|
| Carboxyderivat | Typ | K_{M}/Kᵢ | Kᵢ/Kᵢᵢ |
| Sacch-C6S | UK | - | - |
| Pal-C6'S | K | 0,22 | - |

**Tabelle 11:**

| Inhibierung von SI durch 3'-Aminosaccharose. K_{M}/Kᵢ-Werte sowie die Verhältnisbildung von kompetitiven und unkompetitiven Hemmanteilen | | | | | | | | | |
|---|---|---|---|---|---|---|---|---|---|
| Aminoderivat | SI/Saccharose | | | SI/Maltose | | | SI/Isomaltose | | |
| | Typ | K_{M}/Kᵢ | Kᵢ/Kᵢᵢ | Typ | K_{M}/Kᵢ | Kᵢ/Kᵢᵢ | Typ | K_{M}/Kᵢ | Kᵢ/Kᵢᵢ |
| 3'A-Sacch | NK | 11530 | 1 | NK | 2113 | 1 | K | 8,2 | - |

**Tabelle 12:**

| Inhibierung von GM durch 3'-Aminosaccharose. K_{M}/Kᵢ-Werte sowie die Verhältnisbildung von kompetitiven und unkompetitiven Hemmanteilen | | | |
|---|---|---|---|
| Aminoderivat | Typ | K_{M}/Kᵢ | Kᵢ/Kᵢᵢ |
| 3'A-Sacch | NK | 3,1 | 0,06 |

Die Stärke einer Enzyminhibierung läßt sich aus den Inhibierungskonstanten Kᵢ und Kᵢᵢ ablesen. Der K_{M}/Kᵢ-Wert läßt Aussagen darüber zu, in welchem Verhältnis die Affinität zwischen Enzym und natürlichem Substrat zu der Affinität zwischen Enzym und Inhibitor steht:
Sehr starke Inhibitoren weisen ein Verhältnis K_{M}/Kᵢ > 2 auf.
Starke Inhibitoren weisen ein K_{M}/Kᵢ-Verhältnis zwischen 1 und 2 auf.
Schwache Inhibitoren weisen ein K_{M}/Kᵢ-Verhältnis < 1 auf.

Substanzen ohne jegliche Inhibitorfunktion weisen ein K_{M}/Kᵢ-Verhältnis sehr viel kleiner als 1 auf.

Aus den Tabellen kann entnommen werden, daß Saccharose-C6-Säure und Palatinose-C6'-Säure starke bis sehr starke Inhibitoren der Enzym-/Substrat-Kombinationen SI/Saccharose und SI/Isomaltose sind und auch für die Enzym-/Substrat-Kombination SI/Maltose inhibitorische Wirkung aufweisen. Die Palatinose-C6'-Säure weist auch für die Enzym-/Substrat-Kombination GM/Maltose inhibitorische Wirkung auf. 3'-Aminosaccharose stellt einen starken bis sehr starken Inhibitor für alle untersuchten Enzym-/Substrat-Kombinationen dar.

## Patentansprüche

1. Disaccharidderivat, insbesondere ein Aminoderivat eines Disaccharids, ein oxidiertes Disaccharid oder ein Derivat eines oxidierten Disaccharids zur Behandlung von Hyperglykämien des menschlichen oder tierischen Körpers.

2. Disaccharidderivat nach Anspruch 1, wobei das Aminoderivat des Disaccharids 3'-Aminosaccharose ist.

3. Disaccharidderivat nach Anspruch 1, wobei das oxidierte Disaccharid Saccharose-C6-Säure oder Palatinose-C6'-Säure ist.

4. Disaccharidderivat nach Anspruch 1, wobei das Derivat eines oxidierten Disaccharids ein Amid mit der allgemeinen Strukturformel R'-CO-NHR ist, wobei R'-CO das oxidierte Disaccharid und R=H beziehungsweise R=CₙH₍₂ₙ₊₁₎ mit n = 0-5 ist oder ein Alkylester, insbesondere Methyl- oder Ethylester des oxidierten Disaccharids ist.

5. Arzneimittelzusammensetzung, enthaltend ein Disaccharidderivat, insbesondere ein Aminoderivat eines Disaccharids, ein oxidiertes Disaccharid oder ein Derivat eines oxidierten Disaccharids, zur Vorbeugung oder Behandlung von Hyperglykämien.

6. Arzneimittelzusammensetzung nach Anspruch 5, wobei das Aminoderivat des Disaccharids 3'-Amino-saccharose ist.

7. Arzneimittelzusammensetzung nach Anspruch 5, wobei das oxidierte Disaccharid Saccharose-C6-Säure oder Palatinose-C6'-Säure ist.

8. Arzneimittelzusammensetzung nach Anspruch 5, wobei das Derivat eines oxidierten Disaccharids ein Amid mit der allgemeinen Strukturformel R'-CO-NHR ist, wobei R'-CO das oxidierte Disaccharid und R=H beziehungsweise R=CₙH₍₂ₙ₊₁₎ mit n = 0-5 ist, oder ein Alkylester, insbesondere Methyl- oder Ethylester des oxidierten Disaccharids ist.

9. Lebensmittelzusammensetzung, enthaltend ein Aminoderivat eines Disaccharids, ein Amid eines oxidierten Disaccharids mit der allgemeinen Strukturformel R'-CO-NHR, wobei R'-CO das oxidierte Disaccharid und R=H beziehungsweise R=CₙH₍₂ₙ₊₁₎ mit n = 0-5 ist, ein Methyl- oder Ethylester des oxidierten Disaccharids oder Palatinose-C6'-Säure.

10. Lebensmittelzusammensetzung nach Anspruch 9, wobei das Aminoderivat des Disaccharids 3'-Amino-saccharose ist.

11. In vitro-Verfahren zur Inhibierung von α-Glucosidasen, insbesondere Maltasen oder Saccharasen, wobei eine wässrige, den Glucoamylase/Maltase-Enzymkomplex und/oder den Saccharase/Isomaltase-Enzymkomplex enthaltende Lösung mit ein oder mehreren Disaccharidderivaten, insbesondere Aminoderivaten eines Disaccharids, oxidierten Disacchariden. oder Derivaten eines oxidierten Disaccharides in Kontakt gebracht wird und eine Inhibierung der α-Glucosidase erzielt wird.

12. Verfahren zur Inhibierung von α-Glucosidasen, insbesondere Maltasen oder Saccharasen nach Anspruch 11, wobei das Aminoderivat des Disaccharids. 3'-Aminosaccharose ist.

13. Verfahren zur Inhibierung von α-Glucosidasen, insbesondere Maltasen oder Saccharasen nach Anspruch 11, wobei das oxidierte Disaccharid Saccharose-C6-Säure oder Palatinose-C6'-Säure ist.

14. Verfahren zur Inhibierung von α-Glucosidasen, insbesondere Maltasen oder Saccharasen nach Anspruch 11, wobei das Derivat eines oxidierten Disaccharids ein Amid mit der allgemeinen Strukturformel R'-CO-NHR ist, wobei R'-CO das oxidierte Disaccharid und R=H beziehungsweise R=CₙH₍₂ₙ₊₁₎ mit n = 0-5 ist oder ein Alkylester, insbesondere Methyl- oder Ethylester des oxidierten Disaccharids ist.

15. Verwendung eines Disaccharidderivates, insbesondere eines Aminoderivats eines Disaccharids, eines oxidierten Disaccharids oder eines Derivats eines oxidierten Disaccharids zur Herstellung eines Arzneimittels zur Vorbeugung oder Behandlung von Hyperglykämien.

16. Verwendung eines Disaccharidderivates nach Anspruch 15, wobei das Aminoderivat des Disaccharids 3'-Aminosaccharose ist.

17. Verwendung eines Disaccharidderivates nach Anspruch 15, wobei das oxidierte Disaccharid Saccharose-C6-Säure oder Palatinose-C6'-Säure ist.

18. Verwendung eines Disaccharidderivates nach Anspruch 15, wobei das Derivat eines oxidierten Disaccharids ein Amid mit der allgemeinen Strukturformel R'-CO-NHR ist, wobei R'-CO das oxidierte Disaccharid und R=H beziehungsweise R=CₙH₍₂ₙ₊₁₎ mit n = 0-5 ist oder ein Alkylester, insbesondere Methyl- oder Ethylester des oxidierten Disaccharids ist.

19. Verwendung eines Aminoderivates eines Disaccharides oder eines Amids eines oxidierten Disaccharides mit der allgemeinen Strukturformel R'-CO-NHR, wobei R'-CO das oxidierte Disaccharid und R=H beziehungsweise R=CₙH₍₂ₙ₊₁₎ mit n = 0-5 ist, zur Herstellung eines Lebensmittels zur Vorbeugung oder Behandlung von Hyperglykämien.

20. Verwendung nach Anspruch 19, wobei das Aminoderivat des Disaccharides 3'-Aminosaccharose ist.

## Claims

1. A disaccharide derivative, in particular an amino derivative of a disaccharide, an oxidized disaccharide or a derivative of an oxidized disaccharide for the treatment of hyperglycemias of the human or animal body.

2. The disaccharide derivative as claimed in claim 1, wherein the amino derivative of the disaccharide is 3'-aminosucrose.

3. The disaccharide derivative as claimed in claim 1, wherein the oxidized disaccharide is sucrose-C6-acid or palatinose-C6'-acid.

4. The disaccharide derivative as claimed in claim 1, wherein the derivative of an oxidized disaccharide is an amide of the general structural formula R'-CO-NHR, where R'-CO is the oxidized disaccharide and R = H or R = CₙH₍₂ₙ₊₁₎ where n = 0-5 or an alkyl ester, in particular methyl ester or ethyl ester of the oxidized disaccharide.

5. A drug composition comprising a disaccharide derivative, in particular an amino derivative of a disaccharide, an oxidized disaccharide or a derivative of an oxidized disaccharide for the prevention or treatment of hyperglycemias.

6. The drug composition as claimed in claim 5, wherein the amino derivative of the disaccharide is 3'-aminosucrose.

7. The drug composition as claimed in claim 5, wherein the oxidized disaccharide is sucrose-C6-acid or palatinose-C6'-acid.

8. The drug composition as claimed in claim 5, wherein the derivative of an oxidized disaccharide is an amide of the general structural formula R'-CO-NHR, where R'-CO is the oxidized disaccharide and R = H or R = CₙH₍₂ₙ₊₁₎ where n = 0-5 or an alkyl ester, in particular methyl ester or ethyl ester of the oxidized disaccharide.

9. A food composition comprising an amino derivative of a disaccharide, an amide of an oxidized disaccharide of the general structural formula R'-CO-NHR, where R'-CO is the oxidized disaccharide and R = H or R = CₙH₍₂ₙ₊₁₎ where n = 0-5, a methyl ester or ethyl ester of the oxidized disaccharide or palatinose-C6'-acid.

10. The food composition as claimed in claim 9, wherein the amino derivative of the disaccharide is 3'-aminosucrose.

11. In vitro process for inhibiting α-glucosidases, in particular maltases or sucrases, wherein an aqueous solution comprising the glucoamylase/maltase enzyme complex and/or the sucrase/isomaltase enzyme complex is brought into contact with one or more disaccharide derivatives, in particular amino derivatives of a disaccharide, oxidized disaccharides or derivatives of an oxidized disaccharide and an inhibition of the α-glucosidase is achieved.

12. A process for inhibiting α-glucosidases, in particular maltases or sucrases as claimed in claim 11, wherein the amino derivative of the disaccharide is 3'-aminosucrose.

13. A process for inhibiting α-glucosidases, in particular maltases or sucrases as claimed in claim 11, wherein the oxidized disaccharide is sucrose-C6-acid or palatinose-C6'-acid.

14. A process for inhibiting α-glucosidases, in particular maltases or sucrases as claimed in claim 11, wherein the derivative of an oxidized disaccharide is an amide of the general structural formula R'-CO-NHR, where R'-CO is the oxidized disaccharide and R = H or R = CₙH₍₂ₙ₊₁₎ where n = 0-5 or an alkyl ester, in particular methyl ester or ethyl ester of the oxidized disaccharide.

15. The use of a disaccharide derivative, in particular of an amino derivative of a disaccharide, of an oxidized disaccharide or of a derivative of an oxidized disaccharide for the preparation of a drug for the prevention or treatment of hyperglycemias.

16. The use of a disaccharide derivative as claimed in claim 15, wherein the amino derivative of the disaccharide is 3'-aminosucrose.

17. The use of a disaccharide derivative as claimed in claim 15, wherein the oxidized disaccharide is sucrose-C6-acid or palatinose-C6'-acid.

18. The use of a disaccharide derivative as claimed in claim 15, wherein the derivative of an oxidized disaccharide is an amide of the general structural formula R'-CO-NHR, where R'-CO is the oxidized disaccharide and R = H or R = CₙH₍₂ₙ₊₁₎ where n = 0-5 or an alkyl ester, in particular methyl ester or ethyl ester of the oxidized disaccharide.

19. The use of an amino derivative of a disaccharide or of an amide of an oxidized disaccharide with the general structural formula R'-CO-NHR, where R'-CO is the oxidized disaccharide and R = H or R = CₙH₍₂ₙ₊₁₎ where n = 0-5, for the preparation of a food for the prevention or treatment of hyperglycemias.

20. The use as claimed in claim 19, wherein the amino derivative of the disaccharide is 3'-aminosucrose.

## Revendications

1. Dérivé disaccharidique, notamment un dérivé aminé d'un disaccharide, un disaccharide oxydé ou un dérivé d'un disaccharide oxydé destiné au traitement d'hyperglycémies du corps d'un être humain ou d'un animal.

2. Dérivé disaccharidique selon la revendication 1, **caractérisé en ce que** le dérivé aminé du disaccharide est le 3'-amino-saccharose.

3. Dérivé disaccharidique selon la revendication 1, **caractérisé en ce que** le disaccharide oxydé est le saccharose-C6-acide ou le palatinose-C6'-acide.

4. Dérivé disaccharidique selon la revendication 1, **caractérisé en ce que** le dérivé d'un disaccharide oxydé est un amide qui a pour formule générale R'-CO-NHR, avec R'-CO représentant le disaccharide oxydé et respectivement R=H et R=CₙH₍₂ₙ₊₁₎ avec n = 0-5, ou un alkylester, notamment un ester méthylique ou un ester éthylique du disaccharide oxydé.

5. Composition pharmaceutique comprenant un dérivé disaccharidique, notamment un dérivé aminé d'un disaccharide, un disaccharide oxydé ou un dérivé d'un disaccharide oxydé destiné à la prévention ou au traitement d'hyperglycémies.

6. Composition pharmaceutique selon la revendication 5, **caractérisée en ce que** le dérivé aminé du disaccharide est le 3'-amino-saccharose.

7. Composition pharmaceutique selon la revendication 5, **caractérisée en ce que** le disaccharide oxydé est le saccharose-C6-acide ou le palatinose-C6'-acide.

8. Composition pharmaceutique selon la revendication 5, **caractérisée en ce que** le dérivé d'un disaccharide oxydé est un amide qui a pour formule générale R'-CO-NHR, avec R'-CO représentant le disaccharide oxydé et respectivement R=H et R=CₙH₍₂₊₁₎ avec n = 0-5, ou un alkylester, notamment un ester méthylique ou un ester éthylique du disaccharide oxydé.

9. Composition alimentaire comprenant un dérivé aminé d'un disaccharide, un amide d'un disaccharide oxydé qui a pour formule générale R'-CO-NHR, avec R'-CO représentant le disaccharide oxydé et respectivement R=H et R=CₙH₍₂ₙ₊₁₎ avec n = 0-5, un ester méthylique ou un ester éthylique du disaccharide oxydé ou le palatinose-C6'-acide.

10. Composition alimentaire selon la revendication 9, **caractérisée en ce que** le dérivé aminé du disaccharide est le 3'-amino-saccharose.

11. Procédé in vitro destiné à l'inhibition de α-glucosidases, notamment de maltases ou de saccharases, **caractérisé en ce qu'**on met en contact une solution aqueuse comprenant le complexe enzymatique glucoamylase/maltase et/ou le complexe enzymatique saccharase/isomaltase avec un ou plusieurs dérivés disaccharidiques, notamment des dérivés aminés d'un disaccharide, des disaccharides oxydés ou des dérivés d'un disaccharide oxydé, produisant une inhibition de la α-glucosidase.

12. Procédé destiné à l'inhibition de α-glucosidases, notamment de maltases ou de saccharases selon la revendication 11, **caractérisé en ce que** le dérivé aminé du disaccharide est le 3'-amino-saccharose.

13. Procédé destiné à l'inhibition de α-glucosidases, notamment de maltases ou de saccharases selon la revendication 11, **caractérisé en ce que** le disaccharide oxydé est le saccharose-C6-acide ou le palatinose-C6'-acide.

14. Procédé destiné à l'inhibition de α-glucosidases, notamment de maltases ou de saccharases selon la revendication 11, **caractérisé en ce que** le dérivé d'un disaccharide oxydé est un amide qui a pour formule générale R'-CO-NHR, avec R'-CO représentant le disaccharide oxydé et respectivement R=H et R=CₙH₍₂ₙ₊₁₎ avec n = 0-5, ou un alkylester, notamment un ester méthylique ou un ester éthylique du disaccharide oxydé.

15. Utilisation d'un dérivé disaccharide, notamment un dérivé aminé d'un disaccharide, un disaccharide oxydé ou un dérivé d'un disaccharide oxydé pour la production d'un médicament destiné à la prévention ou au traitement d'hyperglycémies.

16. Utilisation d'un dérivé disaccharide selon la revendication 15, **caractérisée en ce que** le dérivé aminé du disaccharide est le 3'-amino-saccharose.

17. Utilisation d'un dérivé disaccharide selon la revendication 15, **caractérisée en ce que** le disaccharide oxydé est le saccharose-C6-acide ou le palatinose-C6'-acide.

18. Utilisation d'un dérivé disaccharide selon la revendication 15, **caractérisée en ce que** le dérivé d'un disaccharide oxydé est un amide qui a pour formule générale R'-CO-NHR, avec R'-CO représentant le disaccharide oxydé et respectivement R=H et R=CₙH₍₂ₙ₊₁₎ avec n = 0-5, ou un alkylester, notamment un ester méthylique ou un ester éthylique du disaccharide oxydé.

19. Utilisation d'un dérivé aminé d'un disaccharide ou d'un amide d'un disaccharide oxydé qui a pour formule générale R'-CO-NHR, avec R'-CO représentant le disaccharide oxydé et respectivement R=H et R=CₙH(2ₙ₊₁) avec n = 0-5, pour la production d'un aliment destiné à la prévention ou au traitement d'hyperglycémies.

20. Utilisation selon la revendication 19, **caractérisée en ce que** le dérivé aminé du disaccharide est le 3'-amino-saccharose.
